# EUROPÄISCHE PATENTSCHRIFT

(11) **EP 0 633 772 B1**
(45) Veröffentlichungstag und Bekanntmachung des Hinweises auf die Patenterteilung: **02.01.2003**
(21) Anmeldenummer: 93908900.9
(22) Anmeldetag: 02.04.1993
(51) Int. Cl.: A61K 31/52, A61P 9/00

(54) **ARZNEIMITTEL GEGEN HERZ-KREISLAUF-ERKRANKUNGEN**
MEDICAMENT AGAINST CARDIAC CIRCULATORY DISEASES
MEDICAMENT VISANT A REMEDIER AUX MALADIES CARDIOVASCULAIRES

(30) Priorität: 03.04.1992 DE 4211239
(43) Veröffentlichungstag der Anmeldung: 18.01.1995
(73) Patentinhaber: Max-Planck-Gesellschaft zur Förderung der Wissenschaften e.V., D-37073 Göttingen (DE)
(72) Erfinder: PODZUWEIT, Thomas, D-6350 Bad Nauheim (DE)
(74) Vertreter: Weisert, Annekäte, Dipl.-Ing. Dr.-Ing.
(86) Internationale Anmeldenummer: EP9300827
(87) Internationale Veröffentlichungsnummer: WO93019742

(56) Entgegenhaltungen:
- EP-A- 0 463 756
- J.CARDIOVASC.PHARMACOL. Bd. 5, Nr. 6, 1983, Seiten 1040 - 7
- EUR.J.PHARMACOL. Bd. 200, Nr. 1, 1991, Seiten 83 - 7
- J.MED.CHEM. Bd. 18, Nr. II, 1975, Seiten 1117 - 22 in der Anmeldung erw{hnt
- J.PHARMACOL.EXP.THER. Bd. 258, Nr. 3, 1991, Seiten 972 - 8
- J.PHARMACOL.EXP.THER. Bd. 249, Nr. 2, 1989, Seiten 394 - 400
- TIPS REVIEW Bd. 11, 1990, Seiten 150 - 5 in der Anmeldung erw{hnt
- J.MOL.CELL.CARDIOL. Bd. 23, Nr. SPLV, 1991, Seiten ABSTR. - P217 in der Anmeldung erw{hnt

## Beschreibung

Die Erfindung betrifft die Verwendung von Erythro-9-(2-hydroxy-3-nonyl)-adenin bzw. 2-o-Propoxyphenyl-8-azapurin-6-on zur Herstellung eines Arzneimittels gegen Herzrhythmusstörungen, Herzinsuffizienz, Angina pectoris und Bluthochdruck (Hypertonie).

Herz-Kreislauf-Erkrankungen gehören zu den Haupttodesursachen in den Industriestaaten. Ist das Herz nicht mehr imstande, eine den Anforderungen entsprechende Förderleistung zu erbringen, d.h. hat es nicht mehr die Kraft, die dem venösen Angebot entsprechende Blutmenge in die Kreislaufperipherie zu pumpen, so spricht man von Herzinsuffizienz. Man unterscheidet dabei nach dem betroffenen Herzteil eine Rechtsherzinsuffizienz, Linksherzinsuffizienz und beidseitige Insuffizienz (Globalinsuffizienz), je nach dem Schweregrad eine Ruhe- bzw. Belastungsinsuffizienz. Eine Herzinsuffizienz kann mechanische oder biochemische Ursachen haben. Eine mechanisch bedingte Herzinsuffizienz ist möglich durch langdauernde Überbelastung des Myokards infolge erhöhten Widerstands im kleinen oder großen Kreislauf (z.B. bei chronischen Lungenerkrankungen, Mitral- oder Aortenstenose, Hypertonie), durch Ausfall von Herzmuskelfasern bei Myokarditis oder Herzinfarkt, Herzrhythmusstörungen (Tachykardie, Bradykardie) oder Behinderung der Herztätigkeit durch konstriktive Perikarditis oder Herzbeuteltamponade. Eine biochemisch bedingte Herzinsuffizienz tritt auf bei Substratmangel im Herzmuskel infolge ungenügender Durchblutung (Koronarinsuffizienz) oder gestörter Diffusion von den Kapillaren in die Muskelfasern (z.B. bei Myokarditis) und durch mangelhafte Umwandlung von chemischer Energie in mechanische Energie infolge einer Elekrolyt- oder Stoffwechselstörung (Verschiebung des K/Ca-Quotienten, Vitamin-B₁-Mangel, Diabetes mellitus).

Die medikamentöse Therapie der Herzinsuffizienz hat vor allem zum Ziel, die Herzarbeit zu ökonomisieren und die Kontraktionskraft der Herzmuskelfasern zu erhöhen.

Besondere Aufmerksamkeit gilt dabei den Herzrhythmusstörungen, unter denen bereits junge Menschen leiden können, die aber insbesondere für ältere Menschen typisch sind. Die pathologischen Veränderungen, die den Herzrhythmusstörungen unterliegen, beruhen auf einer Störung der Erregungsbildung und/oder der Erregungsleitung. Die Herzfrequenz kann dabei zu hoch (Tachykardie), zu niedrig (Bradykardie) oder unregelmäßig (Arrhythmie) sein.

Kammerflimmern ist oft die Ursache des plötzlichen Herztods. Zunehmende Bedeutung gewinnen biochemische Faktoren in der Genese solcher Arrhythmien. Aber der genaue Ursprung des Kammerflimmerns bleibt spekulativ, und eine wirksame Therapie mit Arzneimitteln ist immer noch nicht bekannt. Gemäß einer Hypothese wird die lokale Kaliumionenkonzentration, die aus dem Herzen freigesetzt wird, mit dem Einsetzen der Arrhythmien und des Kammerflimmerns in Bezug gesetzt, aber der Verlust an Kaliumionen und das Kammerflimmern stehen manchmal in keinem Zusammenhang zueinander. Es wurde vorgeschlagen, daß cyclisches Adenosinmonosphosphat, der Second Messenger der Katecholaminaktivität, einen Bezug zu dem Einsetzen des Kammerflimmerns hat.

Physiologische Mechanismen, die die Synthese oder den Abbau von cAMP in ischaemischen Zellen regulieren, sollten daher für die Entwicklung eines Arzneimittels gegen Kammerflimmern von Bedeutung sein (T. Podzuweit, W.F. Lubbe und L.H. Opie, Lancet i, 341 - 342, 1976).

Bei Schweinen wurde gefunden, daß frühe ventrikuläre Arrhythmien und das Kammerflimmern , die durch Verschlüsse der Koronararterien hervorgerufen wurden, im Zusammenhang mit erhöhten Spiegeln an myokardialem cAMP ebenso wie an Adenosin stehen. Zusätzlich wurde eine Beziehung zwischen den cAMP- und Adenosingehalten des ischaemischen Myokards und der Häufigkeit des Reperfusionskammerflimmerns nachgewiesen. Kammerflimmern, das durch den proximalen Verschluß des Ramus inter ventricularis anterior (Riva) der linken Herzkranzarterie hervorgerufen wurde, wurde durch Vorbehandlung der Schweine mit Atenolol (0,2 bis 1,0 mg/kg i.v.) nicht verhindert.

Cyclische Nukleotide wirken als Second Messenger in der hormonellen Regulation des Zellstoffwechsels. Verschiedene Hormone wirken über ein in der Membran der Rezeptorzelle lokalisiertes Enzymsystem, die Adenylzyklase, welche ATP in cAMP umwandelt. Das cAMP aktiviert eine oder mehrere Proteinkinasen dieser Zelle, welche ihrerseits die ATP-abhängige Phosphorylierung wichtiger Schlüsselenzyme des Intermediärstoffwechsels sowie die Phosphorylierung von membranständigen Proteinen katalysieren. Für die Entstehung der Arrhythmien ist wichtig, daß die Aktivität bestimmter Kalziumkanäle durch eine cAMP-abhängige Phosphorylierung gesteuert wird. Die Phosphorylierung von Ca²⁺-Kanälen (bzw. von einem mit diesen eng assoziierten Protein) durch cAMP-abhängige Proteinkinasen führt zu einer Erhöhung der Öffnungswahrscheinlichkeit dieser Ca²⁺-Kanäle. Die Aufhebung des jeweils durch cAMP provozierten Effekts geschieht durch den wiederum steuerbaren Abbau des cAMPs durch eine spezifische Phosphodiesterase. Analog wird cGMP durch das Enzym Guanylzyklase synthetisiert und durch Phosphodiesterasen abgebaut.

Vier lösliche Phosphodiesterasen wurden aus Extrakten des menschlichen Papillarmuskels unter Verwendung von Anionenaustauschchromatographie isoliert. Diese Enzymaktivitäten wurden als PDE I bis IV nach ihrer Reihenfolge der Elution mit einem ansteigenden Salzgradienten (NaOAc bzw. NaCl) bezeichnet (T. Podzuweit et al., J. Mol. Cell. Cardiol. 23, Supplement V, 1991, Abstract P217).

Obwohl die Primärsequenzen von mehr als 15 verschiedenen cyclische-Nukleotid-Phosphodiesterasen von Säugern bekannt sind, wurde bisher kein Inhibitor, der zwischen Mitgliedern der gleichen Familie unterscheidet, entwickelt (J.A. Beavo und D.H. Reifsnyder, TiPS, Reviews, April 1990, Band 11, Seiten 150 bis 155).

J. Cardiovasc. Pharmacol., 5(6), 1983, 1040-7 beschreibt die Wirkung der Adenosin-Deaminase-Inhibitoren EHNA und 2'-Deoxycoformycin auf die funktionelle und biochemische Erholung des Herzens von einer Ischämie.

Eur. Pharmacol., 200, 1991, 83-7 beschreibt, daß der unselektive PDE-Inhibitor Zaprinast den cGMP-Gehalt der Rattenaorta dosisabhängig erhöht.

J. Pharmacol. Exp. Ther., 258(3), 1991, 972-8 beschreibt das Zusammenwirken von Zaprinast und Aktivatoren der löslichen Guanylatzyklase.

J. Pharmacol. Exp. Ther., 249(2), 1989, 394-400 beschreibt ebenfalls das Zusammenwirken von Zaprinast und Aktivatoren der löslichen Guanylatzyklase.

Die EP-A-0 463 756 beschreibt Inhibitoren der cGMP-Phosphodiesterase zur Behandlung von Herz-Kreislauf-Erkrankungen. Es handelt sich hierbei um neuartige Hemmstoffe, die bevorzugt cGMP-Phosphodiesterasen gegenüber cAMP-Phosphodiesterasen hemmen. Diese Hemmstoffe sind jedoch unselektiv gegenüber den einzelnen cGMP-PDE-Isoenzymen.

Der vorliegenden Erfindung liegt die Aufgabe zugrunde, Inhibitoren bereitzustellen, die verschiedene zyklische Nukleotidphosphodiesterase-Isoenzyme selektiv hemmen können. Insbesondere soll ein Inhibitor für die cGMP-stimulierte Phosphodiesterase (PDE II) zur Verfügung gestellt werden. Erfindungsgemäß soll ein Arzneimittel zur Verfügung gestellt werden, das zur Behandlung von Herz-Kreislauf-Erkrankungen geeignet ist. Weiterhin soll die Verwendung der Inhibitoren für die cGMP-stimulierte Phosphodiesterase (PDE II) für die Bekämpfung und Prophylaxe von Herz-Kreislauf-Erkrankungen und für die Herstellung von Arzneimitteln gegen Herz-Kreislauf-Erkrankungen zur Verfügung gestellt werden. Außerdem sollen Aktivatoren der Guanylzyklase zur Verfügung gestellt werden, durch die die Wirkung der Inhibitoren verstärkt wird.

Überraschenderweise wurde gefunden, daß Inhibitoren der cGMP-stimulierten Phosphodiesterase (PDE II) starke Antiarrhythmika sind, deren Wirkung durch Aktivatoren der Guanylzyklase verstärkt werden kann. Im einzelnen wurde nachgewiesen, daß der bekannte Adenosindeaminaseinhibitor EHNA (Erythro-9-(2-hydroxy-3-nonyl)-adenin) und 2-o-Propoxyphenyl-8-azapurin-6-on starke Antiarrhythmika und selektive Hemmstoffe der cGMP-stimulierten PDE II sind. Die Effekte von EHNA wurden in der Vergangenheit über die Hemmung der Adenosindeaminase erklärt. Die Erfindung zeigt nun erstmals am Beispiel der Arrhythmien, daß die Wirkung von EHNA und von 2-o-Propoxyphenyl-8-azapurin-6-on auf der selektiven Hemmung der cGMP-stimulierten PDE II beruht, eventuell im Zusammenwirken mit der Hemmung der Adenosindeaminase.

Die Erfindung betrifft die Verwendung von Erythro-9-(2-hydroxy-3-nonyl)-adenin als Inhibitor der cGMP-stimulierten Phosphodiesterase (PDE II) zur Herstellung eines Arzneimittels für die Bekämpfung und Prophylaxe von Herzrhythmusstörungen, Herzinsuffizienz, Angina pectoris und Bluthochdruck (Hypertonie). Die Erfindung betrifft ferner die Verwendung von 2-o-Propoxyphenyl-8-azapurin-6-on als Inhibitor der cGMP-stimulierten Phosphodiesterase (PDE II) zur Herstellung von Arzneimitteln für die Bekämpfung und Prophylaxe von Herzrhythmusstörungen und Kammerflimmern. Gegebenenfalls erfolgt die Verwendung zusammen mit einem oder mehreren Aktivatoren der Guanylzyklase.

EHNA ist im Handel erhältlich und kann beispielsweise von Sigma Chemie GmbH, Grünwalder Weg 30, 8024 Deisenhofen bezogen werden.

Das 2-o-Propoxyphenyl-8-azapurin-6-on der folgenden Strukturformel 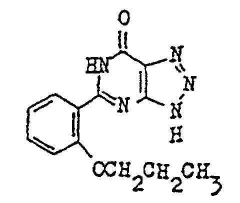 kann nach Literaturangaben hergestellt werden (vgl. beispielsweise B.J. Broughton et al., J. Med. Chem. 18 (II), 1117). Der systematische Name dieser Verbindung lautet 1,4-Dihydro-5-(2-propoxyphenyl-7H-1,2,3-triazolo[4,5-d]pyrimidin-7-on). Die Verbindung ist ein farbloses Pulver und in Wasser nur gering löslich. Sie löst sich in Aceton und Ethanol und in schwach basischen Lösungen. Die Verbindung ist stabil und besitzt niedrige Toxizität. Die akute orale LD₅₀ bei der Maus, der Ratte und dem Kaninchen liegt im Bereich von 1.400 bis 2.000 mg/kg pro Anion.

Als Guanylzyklase-Aktivator können erfindungsgemäß alle solche Verbindungen, von denen diese Aktivität bekannt ist, verwendet werden. Beispiele hierfür sind Nitrite, organische Nitrate, Nitrosoverbindungen und eine Vielzahl anderer Stickstoffoxid enthaltender Substanzen einschließlich Natriumnitroprussid. Diese Verbindungen sind bekannt und werden beispielsweise in Goodman and Gilman's, The Pharmacological Basis of Therapeutics, 7. Auflage, 1985, Seite 798 und insbesondere Seite 806 in dem Kapitel "Drugs used for the treatment of angina: organic nitrates, calcium channel blockers, and β-adrenergic antagonists" beschrieben.

Unter den Aktivatoren der Guanylzyklase sind bevorzugt organische Nitrate wie Nitroglyzerin, Isosorbitdinitrat, Erythrityltetranitrat und Pentaerythrit, Tetranitrat, stickstoffoxidenthaltende Vasodilatatoren, die sogenannten Nitrovasodilatatoren, sowie das atriale natriuretische Peptid (ANP = ANF) und Bradykinin, das EDRF (Endothelium derived relaxing factor) aus der Gefäßwand freisetzt. Dieses EDRF ist identisch mit dem NO-Radikal, das z.B. aus Nitroprussid freigesetzt wird.

Erfindungsgemäß erfolgt die Verabreichung der Inhibitoren alleine oral, beispielsweise in Form von Tabletten, Dragees, Kapseln, Lösungen, oder intraperitoneal, intramuskulär, subkutan, intraartikulär oder intravenös, beispielsweise durch Injektion oder Infusion. Es ist besonders bevorzugt, daß die erfindungsgemäße Verwendung so erfolgt, daß der Wirkstoff verzögert freigesetzt wird, das heißt als Depotformen. Die im folgenden gemachten Ausführungen betreffen die erfindungsgemäße Verwendung. Die erfindungsgemäße Verwendung ist für Herz-Kreislauf-Erkrankungen, insbesondere Herzrhythmusstörungen, Herzinsuffizienz, Myokardischämie, Angina pectoris und Bluthochdruck (Hypertonie) geeignet. Die erfindungsgemäße Verwendung eignet sich zur Unterdrückung ischämiebedingter Arrhythmien,
(1) über primäre antiarrhythmische Wirkung, und
(2) dadurch, daß es eine antiischämische Wirkung hat, d.h. daß es vasodilatierend wirkt.

Weiterhin zeigt die erfindungsgemäße Verwendung keine zu starke positive Inotropie und entlastet dadurch das Herz.

Wegen der vasodilatierenden Wirkung der erfindungsgemäßen Verwendung eignet sich diese auch zur Behandlung der Hypertonie und der Angina pectoris. Wesentlich ist hier die synergistische Wirkung von organischen Nitraten und dem PDE-II-Inhibitor. Die mit Nitraten beobachtete Tachyphylaxie könnte durch EHNA günstig beeinflußt werden.

Einheitsdosen können beispielsweise 1 bis 4 mal täglich verabreicht werden. Die exakte Dosis hängt vom Verabreichungsweg und dem zu behandelnden Zustand ab. Naturgemäß kann es erforderlich sein, Routinevariationen der Dosis je nach dem Alter und dem Gewicht des Patienten sowie der Schwere des zu behandelnden Krankheitszustandes vorzunehmen.

Bei der erfindungsgemäßen Verwendung ohne Aktivatoren der Guanylzyklase können EHNA oder Zaprinast in an sich bekannter Weise unter Verwendung eines oder mehrerer pharmazeutisch annehmbarer Träger oder Verdünnungsmittel formuliert werden. Die Zubereitungen können für die orale Verabreichung oder in einer für die Verabreichung durch Injektion oder Infusion geeigneten Weise formuliert werden.

Werden erfindungsgemäß ein PDE-II-Inhibitor und ein Aktivator der Guanylzyklase verwendet, kann die Verwendung gleichzeitig, getrennt oder zeitlich abgestuft erfolgen. Beispielsweise kann das Arzneimittel gleichzeitig einen PDE-II-Inhibitor und einen Aktivator der Guanylzyklase enthalten. Es ist jedoch auch möglich, daß das Arzneimittel nur den PDE-II-Inhibitor enthält, und daß der Aktivator der Guanylzyklase getrennt, gleichzeitig oder zeitlich abgestuft verabreicht wird. Die Verabreichungswege für den Inhibitor und den Aktivator der Guanylzyklase können sich unterscheiden. Beispielsweise kann der Inhibitor subkutan oder durch Injektion oder Infusion verabreicht werden, und der Aktivator der Guanylzyklase kann beispielsweise durch Inhalation oder als Spray verabreicht werden. Der Inhibitor und der Aktivator der Guanylzyklase können somit jeweils auf unterschiedliche Weise kombiniert sein.

Die pharmazeutischen Zubereitungen für die orale Verabreichung können in Form von beispielsweise Tabletten oder Kapseln, die nach an sich bekannten Verfahren mit pharmazeutisch annehmbaren Verdünnungsmitteln, wie Bindemitteln (zum Beispiel vorgelatinierter Maisstärke, Polyvinylpyrrolidon oder Hydroxypropylmethylcellulose), Füllstoffen (zum Beispiel Lactose, Saccharose, Mannit, Maisstärke, mikrokristalline Cellulose oder Calciumhydrogenphosphat); Schmiermitteln (zum Beispiel Stearinsäure, Polyethylenglykol, Magnesiumstearat, Talk oder Siliciumdioxid); Desintegrationsmitteln (zum Beispiel Kartoffelstärke, Natriumstärkeglykolat oder Natriumcarboxymethylcellulose); oder Benetzungsmitteln (zum Beispiel Natriumlaurylsulfat), hergestellt werden, vorliegen. Die Tabletten können nach an sich bekannten Verfahren überzogen werden. Flüssige Zubereitungen für die orale Verabreichung können in Form von beispielsweise wäßrigen oder öligen Lösungen, Sirupen, Elixieren, Emulsionen oder Suspensionen vorliegen, oder sie können als Trockenprodukt für die Konstitution mit Wasser oder einem anderen geeigneten Träger vor der Verwendung vorliegen. Solche flüssigen Zubereitungen können nach an sich bekannten Verfahren mit pharmazeutisch annehmbaren Zusatzstoffen hergestellt werden, wie Suspensionsmitteln (zum Beispiel Sorbitsirup, Cellulosederivaten, Glucose/Zucker-Sirup, Gelatine, Aluminiumstearatgel oder hydrierten genießbaren Fetten); Emulgiermitteln (zum Beispiel Lecithin, Gummi arabicum oder Sorbitan-monooleat); nichtwäßrigen Trägern (zum Beispiel Mandelöl, öligen Estern, Ethylalkohol oder fraktionierten Pflanzenölen); und Konservierungsmitteln (zum Beispiel Methyl- oder Propyl-p-hydroxybenzoaten oder Sorbinsäure). Die flüssigen Zubereitungen können auch an sich bekannte Puffer, Geschmacks- bzw. Aromamittel, Farbstoffe und Süßstoffe, je nach Bedarf, enthalten.

Für die parenterale Verabreichung können die Verbindungen für Injektion, bevorzugt intravenöse, intramuskuläre oder subkutane Injektion oder Infusion formuliert werden. Zubereitungen für die Injektion können in Eindosenform, zum Beispiel in Ampullen, oder in Mehrfachdosis-Behältern mit einem zugegebenen Konservierungsstoff vorliegen. Die Zubereitungen können in Form von Suspensionen, Lösungen oder Emulsionen in öligen oder wäßrigen Trägern vorliegen und Zubereitungshilfsmittel enthalten, wie Suspendier-, Stabilisier- und/oder Dispersionsmittel, und/oder Mittel zur Einstellung der Tonizität der Lösung.

Methylxanthine besitzen sowohl als cyclische Nukleotid-Phosphodiesterase(PDE)-Inhibitoren als auch Adenosin-Rezeptorantagonisten eine lange Geschichte. Der Mangel an Selektivität dieser Verbindungen legt eine Beziehung zwischen Strukturdomänen des Adenosin-Rezeptors und der Phosphodiesterase-Isoenzyme nahe. Die vorliegenden Daten erstrecken die bestehenden Ähnlichkeiten auf das Enzym Adenosindeaminase (Adenosinaminohydrolase, EC 3.5.4.4) (ADA), indem sie zeigen, daß der synthetische Adenosindeaminase-Inhibitor Erythro-9-(2-hydroxy-3-nonyl)-adenin (EHNA) auch ein starker, und soweit lösliche Phosphodiesterasen betroffen sind, selektiver Inhibitor der cGMP-stimulierten PDE-II-Isoenzyme aus Schweine- und Menschen-Myokard ist. Da sich lösliche und membranständige PDE-Isoenzyme in ihren Eigenschaften sehr ähneln, ist mit Sicherheit zu erwarten, daß die an löslichen Phosphodiesterasen gewonnenen Ergebnisse auch für die membranständigen Enzyme gelten.

### MATERIAL UND METHODEN

EHNA-HCl wurde von Burroughs - Wellcome Co. (Greenville, NC, USA) bezogen. c[8-³H]AMP und c[8-³H]GMP wurden vom Radiochemical Centre (Amersham, UK) bezogen. Calmodulin stammte von Boehringer (Mannheim, BRD) und DEAE Sepharose CL-6B und Papaverin stammten von Sigma (Deisenhofen, BRD). Alle anderen Chemikalien stammten von Merck (Darmstadt, BRD) und waren mindestens analysenrein. Hochreines Wasser wurde mit dem Milli-Q-Wasserreinigungssystem (Millipore Corp., Eschborn, BRD) erzeugt. Frische Stammlösungen der Inhibitoren wurden unter gedämpftem Licht mindestens täglich hergestellt.

### Quelle des Gewebes, Extraktion und Chromatographie

Proben der linken ventrikulären Papillar-Muskeln wurden mit Zustimmung der Patienten, die sich einem Austausch einer Mitralklappe im Universitäts-Krankenhaus Gießen, BRD, unterzogen, erhalten. Die Myokard-Proben vom Schwein (männliche Schweine der Deutschen Landrasse, 25 bis 30 kg Körpergewicht) wurden nach Exzision der Herzen unter Nembutal-Anaesthesie erhalten. Die Proben wurden sofort in flüssigem Stickstoff eingefroren und wurden dann in einer Hochgeschwindigkeitsmühle pulverisiert und in flüssigem Stickstoff vorgekühlt. Das gefrorene Gewebspulver wurde durch ein Sieb aus rostfreiem Stahl (-196°C) (Porengröße: 0,315 mm) in einen Homogenisierungspuffer (4°C) (1 ml pro 100 mg Pulver), bestehend aus (Endkonzentrationen): 20 mM Bis-Tris, 2 mM EDTA, 5 mM 2-Mercaptoethanol, 2 mM Benzamidin, 50 µM Phenylmethylsulfonylfluorid, 50 mM Natriumacetat, pH 6,5, gegeben. Alle folgenden Schritte wurden bei 4°C durchgeführt. Das Homogenat wurde 30 Minuten bei 48.000 g zentrifugiert. 50 ml des Überstands wurden entnommen und auf eine Säule (20 x 1,6 cm) aus DEAE-Sepharose CL-6B, die mit Homogenisierungspuffer vorequilibriert worden war, geladen. Die Säule wurde mit 200 ml Puffer gewaschen, und die PDE-Aktivitäten wurden mit einem linearen Gradienten von 0,05 bis 1 M Natriumacetat in Puffer (Flußzeit des Gradienten: 500 min) eluiert. Die Flußrate betrug 1 ml/min. 5-ml-Fraktionen wurden gewonnen und auf PDE-Aktivität untersucht.

### Test auf die PDE-Aktivität und Bestimmung der IC₅₀-Werte

Die PDE-Aktivität wurde unter Verwendung von HPLC mit einer Off-Line-Flüssig-Szintillationsspektrometrie gemessen. Das Reaktionsgemisch bestand aus (Endkonzentrationen): 1 µM c[8-³H] AMP oder 1 µM c[8-³H]GMP (9,25 GBq/mmol) (ungefähr 35.000 cpm), 1 µM cGMP (sofern vorhanden), 5 mM MgCl₂, 5 x 10⁻⁷M bis 10⁻³M Inhibitor (sofern anwendbar) und 20 mM Tris-HCl, pH 7,5, in einem Gesamtvolumen von 200 µl. Die Enzymaktivität wurde bei 25°C mit 10minütigen Inkubationen gemessen. Die Reaktionen wurden durch Zugabe von 50 µl Enzymlösung gestartet und durch Injektion von 20 µl 60%ige Perchlorsäure beendet. Aliquote zu 20 µl der sauren Überstände wurden angesaugt und in ein automatisiertes HPLC-System (Säule LiChrospher RP-18e) (250 x 4 mm; 5 µm Packung) injiziert. Die radioaktiven Substrate und die Produkte der PDE-Reaktion wurden unter Verwendung eines RACKBETA 1219-Flüssig-Szintillationszählers (LKB Wallac, Freiburg, BRD) quantitativ bestimmt. Die IC₅₀-Werte (Konzentrationen mit 50%iger Inhibierung) wurden bei 1 µM cAMP oder cGMP unter Verwendung der Peak-Fraktionen bestimmt. Die Daten wurden mit Hilfe der sigmoidalen logistischen Funktion mit vier Parametern gefittet.

### ERGEBNISSE

Die löslichen PDE-Isoenzyme aus Schweine- und Menschen-Myokard wurden durch Anionenaustauschchromatographie unter Verwendung von DEAE-Sepharose CL-6B aufgetrennt. Die Enzymaktivitäten wurden mit PDE I bis IV gemäß der Nomenklatur nach J.A. Beavo und D.H. Reifsnyder bezeichnet. Die PDE II-Aktivität wurde durch cGMP (1 µM) stimuliert. Das Enzym wurde dosisabhängig durch 2-o-Propoxyphenyl-8-azapurin-6-on [IC₅₀: 8 x 10⁻⁶M (PDE I), 3 x 10⁻⁵M (PDE II)] inhibiert.

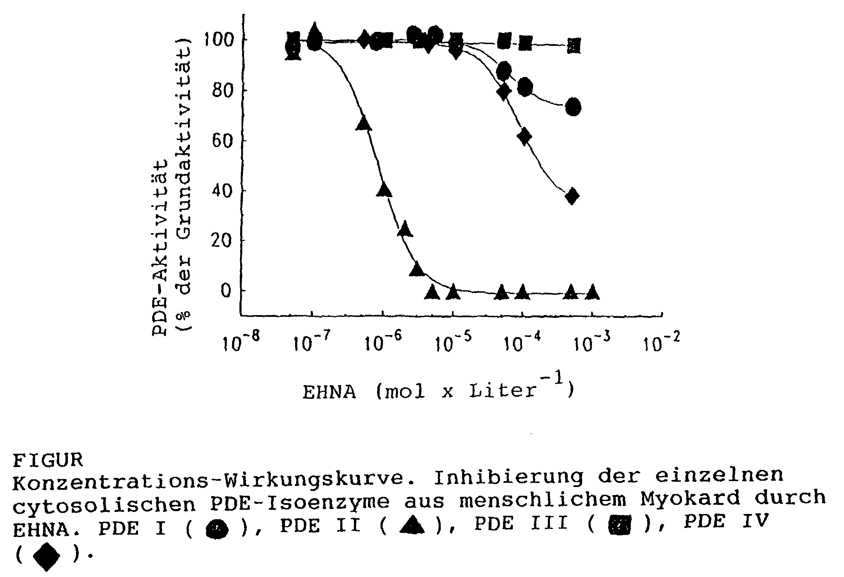

Jedoch fehlte dem Schweine-Myokard die Ca²⁺-Calmodulinstimulierte lösliche PDE I-Aktivität. EHNA zeigte eine konzentrationsabhängige Inhibierung des cytosolischen cGMP-stimulierten PDE II-Isoenzyms aus menschlichem Myokard (IC₅₀ = 8 x 10⁻⁷M). Bezogen auf die anderen löslichen PDE-Isoenzyme war die Inhibierung des cGMP-stimulierten PDE II-Subtyps selektiv und in hohem Maße konzentrationsabhängig. Die Inhibierung der PDE-Isotypen I, III und IV begann bei einer Konzentration an EHNA, die das cGMP-stimulierte Enzym zu mehr als 99% inhibierte. Die erhaltenen Ergebnisse sind in der beigefügten Figur dargestellt.

Die Ergebnisse zeigen, daß EHNA als Inhibitor des Enzyms aktiver als der nichtselektive PDE-Inhibitor Papaverin (IC₅₀ = 2 x 10⁻⁴M) auf einer molaren Basis war. Es wurde auch gefunden, daß EHNA die lösliche cGMP-stimulierte PDE II aus Schweine-Myokard dosisabhängig (IC₅₀ = 2 x 10⁻⁶M) inhibierte. Die Inhibierung der anderen cyclische-Nukleotid-Phosphodiesterasen aus Schweine-Myokard war vernachlässigbar (IC₅₀ > 100 µM).

### DISKUSSION

Bis jetzt wurden vier lösliche cyclische-Nukleotid-Phosphodiesterase-Isoenzyme aus Säuger-Myokard unter Verwendung von Anionenaustauschchromatographie aufgetrennt. Diese Enzyme können nach ihren regulatorischen Eigenschaften, d.h. der Aktivierung durch cGMP oder Ca²⁺-Calmodulin oder die Inhibierung durch cGMP oder pharmakologische Inhibitoren unterschieden werden. Selektive Inhibitoren waren für die Ca²⁺-Calmodulin-stimulierte PDE I, die cGMP-inhibierte PDE III und die Rolipram-sensitive PDE IV verfügbar. Jedoch waren bis jetzt selektive Inhibitoren der cGMP-stimulierten Isoenzyme nicht verfügbar. In der vorliegenden Anmeldung wird erstmals gezeigt, daß der reversible Adenosindeaminase-Inhibitor EHNA ein starker Inhibitor der cytosolischen PDE II aus Schweine- und Menschen-Myokard ist. Bezogen auf die anderen löslichen PDE's war die Inhibierung selektiv für das cGMP-stimulierte Isoenzym (vgl. die Figur) mit einem IC₅₀-Wert von 8 x 10⁻⁷M (Mensch) oder 2 x 10⁻⁶M (Schwein). Diese Daten zeigen strukturelle Ähnlichkeiten der katalytischen und/oder regulatorischen Domänen der zwei offensichtlich nicht verwandten Enzyme Adenosindeaminase und cGMP-stimulierte cyclische-Nukleotid-Phosphodiesterase.

Adenosindeaminase katalysiert die Desaminierung von Adenosin, 2'-Desoxyadenosin und von verschiedenen der anderen cytotoxischen Adenosin-Analogen. Kompetitiv gehemmt wird dieses Enzym durch die synthetische Verbindung Erythro-9-(2-hydroxy-3-nonyl)-adenin (EHNA). Bei EHNA ist der Riboserest von Adenosin durch eine aliphatische Kette ersetzt. Die Inhibierung von ADA erfolgt in zwei Stufen, bei denen das 6-NH₂-Desaminierungsziel von EHNA nicht betroffen ist. Der anfängliche Bindungsschritt ist schnell und gehorcht einer klassischen kompetitiven Inhibierung (Kᵢ = 2 x 10⁻⁷M), was wahrscheinlich mit einer Bindung der Nonyl-Seitenkette an eine hydrophobe Region in Nachbarschaft zu der katalytischen Stelle von ADA verbunden ist. Dann erfolgt eine anschließende Umordnung entweder des Inhibitors oder des Enzyms, was zu einem festen Enzym-Inhibitor-Komplex (gesamte Inhibierungskonstante 1,7 x 10⁻⁹M) führt, aus dem der Inhibitor nur langsam abdissoziiert. Die Inhibierungskonstante des ersten Bindungsschritts war niedriger als der Kₘ-Wert für das Substrat Adenosin (Kₘ = 2 x 10⁻⁵M), aber war überraschenderweise vergleichbar dem IC₅₀-Wert für die Inhibierung der cGMP-stimulierten PDE in der vorliegenden Untersuchung. Folglich besitzen Dosen von größer als 10⁻⁷M EHNA inhibierende Wirkungen auf beide Enzyme.

Im Handel erhältliches EHNA, das bei der vorliegenden Untersuchung verwendet wurde, ist ein racemisches Gemisch aus Erythro-(+)-9-(2S-hydroxy-3R-nonyl)-adenin und Erythro-(-)-9-(2R-hydroxy-3S-nonyl)-adenin [(±)-EHNA]. Der Kᵢ-Wert von (±)-EHNA bei menschlicher ADA wurde zu 4 nM bestimmt, was etwa dem 2fachen Wert von (+)-(2S,3R)-EHNA entspricht. Jedoch wurden viel höhere Konzentrationen an EHNA tatsächlich benötigt, um ADA in intakten Zellen zu inhibieren.

Die cGMP-stimulierte PDE ist eines von mindestens zwei löslichen Isoenzymen, die die beiden Second Messenger-Moleküle, cAMP und cGMP, spalten. Die Fähigkeit dieses Enzyms, cAMP oder cGMP zu hydrolysieren, wird in Gegenwart von cGMP infolge allosterischer Aktivierung des Enzyms erhöht, wodurch es zu einem Rezeptor für cGMP wird. Jedoch ist die genaue physiologische Funktion dieses Enzyms immer noch nicht bekannt. Die vorliegenden Ergebnisse legen eine wichtige Rolle der cGMP-stimulierten PDE bei der Kontrolle des Herzrhythmus nahe.

PDE-II-Inhibitoren wie EHNA erweisen sich als Prototyp-Arzneimittel mit inhibierenden Wirkungen sowohl auf Adenosindeaminase als auch auf cGMP-stimulierte PDE. Im Gegensatz dazu ist 2-o-Propoxyphenyl-8-azapurin-6-on ein selektiver PDE-Hemmstoff, der die Adenosindeaminase nicht inhibiert.

Die folgenden Beispiele erläutern die Erfindung.

### Beispiel 1

### Tabletten für die orale Verabreichung

### A. Direkte Kompression

(1)

| | |
|---|---|
| Wirkstoff: EHNA | 2 mg/Tablette |
| Magnesiumstearat BP | 0,65 mg/Tablette |
| wasserfreie Lactose | 80 mg/Tablette |

Der Wirkstoff wird mit der wasserfreien Lactose und dem Magnesiumstearat vermischt, und das Gemisch wird gesiebt. Das entstehende Gemisch wird zu Tabletten unter Verwendung einer Tablettiermaschine verpreßt.
Die gleiche Formulierung kann auch mit 2-o-Propoxyphenyl-8-azapurin-6-on als Wirkstoff vorgenommen werden. Gegebenenfalls wird ein Aktivator der Guanylzyklase hinzugefügt.
(2)

| | |
|---|---|
| Wirkstoff: EHNA | 2,5 mg/Tablette |
| Magnesiumstearat BP | 0,7 mg/Tablette |
| mikrokristalline Cellulose NF | 100 mg/Tablette |

Der Wirkstoff wird gesiebt und mit der mikrokristallinen Cellulose und dem Magnesiumstearat vermischt. Das entstehende Gemisch wird unter Verwendung einer Tablettiermaschine zu Tabletten verpreßt.

Die gleiche Formulierung kann auch mit 2-o-Propoxyphenyl-8-azapurin-6-on als Wirkstoff vorgenommen werden. Gegebenenfalls wird ein Aktivator der Guanylzyklase hinzugefügt.

### B. Nasse Granulierung

| | |
|---|---|
| Wirkstoff: EHNA | 30,0 mg/Tablette |
| Lactose BP | 150,0 mg/Tablette |
| Stärke BP | 30,0 mg/Tablette |
| vorgelatinierte Maisstärke BP | 15,0 mg/Tablette |
| Magnesiumstearat BP | 1,5 mg/Tablette |

Der Wirkstoff wird durch ein geeignetes Sieb gesiebt und mit der Lactose, der Stärke und der vorgelatinierten Maisstärke vermischt. Geeignete Volumina an gereinigtem Wasser werden zugegeben, und das Pulver wird granuliert. Nach dem Trocknen wird das Granulat gesiebt und mit dem Magnesiumstearat vermischt. Das Granulat wird dann unter Verwendung von Lochstanzen mit geeignetem Durchmesser zu Tabletten verpreßt.

Die gleiche Formulierung kann auch mit 2-o-Propoxyphenyl-8-azapurin-6-on als Wirkstoff vorgenommen werden. Gegebenenfalls wird ein Aktivator der Guanylzyklase hinzugefügt.

Tabletten anderer Zusammensetzung können hergestellt werden, indem man das Verhältnis von Wirkstoff zu Lactose oder das Kompressionsgewicht ändert und entsprechende Lochstanzen verwendet.

### Beispiel 2

### Kapseln

| | |
|---|---|
| Wirkstoff: EHNA | 30 mg/Tablette |
| freifließende Stärke | 150 mg/Tablette |
| Magnesiumstearat BP | 1 mg/Tablette |

Der Wirkstoff wird gesiebt und mit den anderen Bestandteilen vermischt. Das Gemisch wird unter Verwendung einer geeigneten Vorrichtung in Hartgelatinekapseln Nr. 2 gefüllt. Andere Kapseln können hergestellt werden, indem man das Füllgewicht ändert und erforderlichenfalls die Kapselgröße entsprechend ändert.

Die gleiche Formulierung kann auch mit 2-o-Propoxyphenyl-8-azapurin-6-on als Wirkstoff vorgenommen werden. Gegebenenfalls wird ein Aktivator der Guanylzyklase hinzugefügt.

### Beispiel 3

### Injektion für intravenöse Verabreichung

| | |
|---|---|
| Wirkstoff: EHNA | 1,5 - 3,0 mg/ml |
| Natriumchlorid-intravenöse Infusion, BP, 0,9 % Gew./Vol. | 1 ml |
| Ansatzgröße | 2500 ml |

Der Wirkstoff wird in einem Teil der Natriumchlorid-intravenösen Infusion gelöst, die Lösung mit der Natriumchloridintravenösen Infusion auf das Volumen eingestellt und die Lösung gründlich vermischt. Die Lösung wird in klare, Typ 1, 10-ml-Glasampullen eingefüllt und unter Stickstoff im Kopfraum durch Abschmelzen des Glases abgesiegelt. Die Ampullen werden durch Erhitzen im Autoklaven bei 120°C für nicht kürzer als 20 Minuten sterilisiert.

## Patentansprüche

1. Verwendung von Erythro-9-(2-hydroxy-3-nonyl)-adenin als Inhibitor der cGMP-stimulierten Phosphodiesterase (PDE II) zur Herstellung eines Arzneimittels für die Bekämpfung und Prophylaxe von Herzrhythmusstörungen, Herzinsuffizienz, Angina pectoris und Bluthochdruck.

2. Verwendung nach Anspruch 1, **dadurch gekennzeichnet, daß** die Verwendung zusammen mit einem oder mehreren Aktivatoren der Guanylzyklase erfolgt, wobei die Verwendung gleichzeitig, getrennt oder zeitlich abgestuft erfolgen kann.

3. Verwendung nach mindestens einem der Ansprüche 1 oder 2, **dadurch gekennzeichnet , daß** für die orale, intraperitoneale, intramuskuläre, subkutane, intraartikuläre oder intravenöse Verabreichung geeignete Arzneimittel hergestellt werden.

4. Verwendung nach mindestens einem der Ansprüche 1 bis 3, **dadurch gekennzeichnet, daß** Tabletten, Dragees, Kapseln, Lösungen oder für die Injektion oder Infusion geeignete Präparate hergestellt werden.

5. Verwendung von 2-o-Propoxyphenyl-8-azapurin-6-on als Inhibitor der cGMP-stimulierten Phosphodiesterase (PDE II) zur Herstellung eines Arzneimittels für die Bekämpfung und Prophylaxe von Arrhythmien und Kammerflimmern.

6. Verwendung nach Anspruch 5, **dadurch gekennzeichnet, daß** die Verwendung zusammen mit einem oder mehreren Aktivatoren der Guanylzyklase erfolgt, wobei die Verwendung gleichzeitig, getrennt oder zeitlich abgestuft erfolgen kann.

7. Verwendung nach mindestens einem der Ansprüche 5 oder 6, **dadurch gekennzeichnet, daß** für die orale, intraperitoneale, intramuskuläre, subkutane, intraartikuläre oder intravenöse Verabreichung geeignete Arzneimittel hergestellt werden.

8. Verwendung nach mindestens einem der Ansprüche 5 bis 7, **dadurch gekennzeichnet, daß** Tabletten, Dragees, Kapseln, Lösungen oder für die Injektion oder Infusion geeignete Präparate hergestellt werden.

## Claims

1. Use of erythro-9-(2-hydroxy-3-nonyl)-adenine as inhibitor of the cGMP stimulated phosphodiesterase (PDE II) for producing a medicine for the combating and prophylaxis of disturbances of the cardiac rhythm, cardiac insufficiency, angina pectoris and hypertension.

2. Use according to claim 1, **characterised in that** use takes place together with one or more activators of guanylic cyclase, wherein use may be simultaneous, separate or temporally graduated.

3. Use according to at least one of claims 1 or 2, **characterised in that** suitable medicines are produced for oral, intraperitoneal, intramuscular, subcutaneous, intraarticular or intravenous administration.

4. Use according to at least one of claims 1 to 3, **characterised in that** tablets, pills, capsules, solutions or preparations suitable for injection or infusion are produced.

5. Use of 2-o-propoxyphenyl-8-azapurine-6-on as inhibitor of the cGMP stimulated phosphodiesterase (PDE II) for producing a medicine for the combating and prophylaxis of arrhythmias and ventricular fibrillations.

6. Use according to claim 5, **characterised in that** use takes place together with one or more activators of the guanylic cyclase, wherein use may be simultaneous, separate or temporally graduated.

7. Use according to at least one of claims 5 or 6, **characterised in that** suitable medicines are produced for oral, intraperitoneal, intramuscular, subcutaneous, intraarticular dr intravenous administration.

8. Use according to at least one of claims 5 to 7, **characterised in that** tablets, pills, capsules, solutions or preparations for injection or infusion are produced.

## Revendications

1. Utilisation de l'érythro-9-(2-hydroxy-3-nonyl)-adénine comme inhibiteur de la phosphodiestérase (PDE II) stimulée par la GMPc pour préparer un médicament destiné à combattre et prévenir les troubles du rythme cardiaque, l'insuffisance cardiaque, l'angor et l'hypertension sanguine.

2. Utilisation selon la revendication 1, **caractérisée en ce que** l'utilisation se fait ensemble avec un ou plusieurs activateurs de la guanyle cyclase, dans laquelle l'utilisation peut se faire de manière simultanée, séparée ou étagée dans le temps.

3. Utilisation selon l'une au moins des revendications 1 ou 2, **caractérisée en ce que** l'on prépare des médicaments qui conviennent pour une administration par voie orale, intrapéritonéale, intramusculaire, sous-cutanée, intra-artioulaire ou intraveineuse.

4. Utilisation selon l'une au moins des revendications 1 à 3, **caractérisée en ce que** l'on prépare des comprimés, des dragées, des gélules, des solutions ou bien des préparations qui conviennent pour l'injection ou la perfusion.

5. Utilisation de la 2-o-propoxyphényl-5-azapurin-6-one comme inhibiteur de la phosphodiestérase (PDE II) stimulée par la GMPc pour préparer un médicament destiné à combattre et prévenir les arythmies et la fibrillation ventriculaire.

6. Utilisation selon la revendication 6, **caractérisée en ce que** l'utilisation se fait ensemble avec un ou plusieurs activateurs de la guanyle cyclase, dans laquelle l'utilisation peut se faire de manière simultanée, séparée ou étagée dans le temps.

7. Utilisation selon l'une au moins des revendications 5 ou 6, **caractérisée en ce que** l'on prépare des médicaments qui conviennent pour une administration par voie orale, intrapéritonéale, intramusculaire, sous-cutanée, intra-articulaire ou intraveineuse.

8. Utilisation selon l'une au moins des revendications 5 à 7, **caractérisée en ce que** l'on prépare des comprimés, des dragées, des gélules, des solutions ou bien des préparations qui conviennent pour l'injection ou la perfusion.
